# EUROPEAN PATENT SPECIFICATION

(11) **EP 2 662 113 B1**
(45) Date of publication and mention of the grant of the patent: **31.08.2016**
(21) Application number: 13164437.9
(22) Date of filing: 19.04.2013
(51) Int. Cl.: A61N 1/378, A61B 5/00, A61M 25/00, A61N 1/372, A61B 5/042, A61N 1/362

(54) **Leadless heart stimulation and/or monitoring device**
Leitungslose Herzstimulations- und/oder -überwachungsvorrichtung
Stimulation cardiaque sans fil et/ou dispositif de contrôle

(30) Priority: 08.05.2012 US 201261643912 P; 08.05.2012 US 201261643911 P
(43) Date of publication of application: 13.11.2013
(73) Proprietor: BIOTRONIK SE & Co. KG, 12359 Berlin (DE)
(72) Inventor: Taff, Brian, Portland, OR Oregon 97239 (US); von Arx, Jeffrey, Lake Oswego, OR Oregon 97035 (US); Kraetschmer, Hannes, West Linn, OR Oregon 97068 (US); Muessig, Dirk, West Linn, OR 97068 (US); Whittington, R. Hollis, Portland, OR 97202 (US); Moulder, J. Christopher, Portland, OR 97202 (US); Klein, Harold, Tualatin, OR 97602 (US); Smith, Kerry, Wilsonville, OR 97070 (US)
(74) Representative: Galander, Marcus

(56) References cited:
- WO-A1-2006/045075
- WO-A1-2006/065394
- WO-A2-00/16686
- WO-A2-2006/020370
- WO-A2-2008/034005
- US-A1- 2002 077 556
- US-A1- 2011 160 557
- US-A1- 2012 095 539

## Description

The invention refers to an leadless heart stimulation and/or monitoring device such as an leadless pacemaker. The device comprises a sealed housing, one or more electrodes configured to electrically contact heart tissue when in use and electric components arranged within the housing. The electric components are at least in part operationally connected to the at least one electrode. Further, the electric components comprise a power supply for providing power supply to at least some of the other electric components.

WO2006/065394A1 discloses an intra-thoracic cardiac stimulation device, while WO2008/034005A2 discloses a leadless electrode for use with an implantable cardiac pacemaker.

In recent years, earnest efforts have been undertaken to develop key components for realizing leadless pacemaker designs. Such engagements have centered on weaning the delivery of pacing waveforms from explicit, wired linkages to distally-stationed pulse generation units.

The strategy, in recognition of the notable fraction of implant complications stemming from lead-associated factors, has crafted a unique set of development criteria for affiliated pacemaker designs. Most proposed configurations have explored myocardial interfacing through intravenous implantation. After injection via a catheter the device resides within targeted heart chamber. The sizing constraints presented by such placements, coupled with the challenges linked to device extraction and replacement, have fostered demand for both higher energy density primary cell chemistries and rechargeable in-implant power sources. It is an object of the invention to provide such a cardiac device that meets the demand at least in part.

According to the invention, this object is achieved by an leadless heart stimulation and/or monitoring system comprising a device having a sealed housing, one or more electrodes configured to electrically contact heart tissue when in use and electric components arranged within the housing. The electric components are at least in part operationally connected to the at least one electrode. The electric components include a power supply for providing power to said electric components. The power supply includes a rechargeable battery and further comprises an implant-based coil that is configured to receive electric power via a tuned magnetic field.

The system further comprises a catheter having a distal end and a catheter-based coil arranged at or near said distal end. The catheter is dimensioned to be introduced into a heart chamber and the catheter-based coil is tuned to said implant-based coil.

Thus, means are provided for renewing on-board system power in leadless pacemakers. By means of an infrequent (once every few years; preferred about five years) venous access procedure, a specially-designed catheter can interact with the implant which is anchored to the myocardium and reside within the patient's heart in ways that maximize electromagnetic coupling dynamics and provide optimal recharging responses.

The invention includes the recognition that a key complication for rechargeable support arises from the complexities associated with delivering effective wireless coupling from electromagnetic sources outside of the body to devices stationed within it. In even the most efficient telemetry interfacing systems, inductive charging efficiencies notably decay as a function of the receiving unit's implant depth. In general the voltage across an inductive link falls of as the cube of the distance separating the coils, which means that the power transferred falls off to the sixth power of the distance. The coil size associated with the shallow pockets of traditional pacemakers already overwhelms the available volumes presented by packaging constraints endemic to pacer configurations and thus has little opportunity for effective scaling. With this context in effect, the invention provides a solution for circumventing the efficiency losses associated with inductive charging from sources outside the body by describing a technique where venous catheter-based interfacing serves to maximize the recharging response.

According to a preferred embodiment, the at least one electrode is configured to anchor the device in heart tissue and serves a fixation element. It is therefore preferred if the at least one electrode is a helical screw-in electrode.

Preferably, the electric components comprise a battery recharging circuit that is electrically connected to both, the implant-based coil and the rechargeable battery of the power supply. The housing of the device preferably has a diameter of less than 8 mm and even more preferred, less than 6,3 mm (19 French).

The device comprises a second fixation element that is arranged at a proximal end and configured to be latched by a capturing element of a catheter. In this context, it is preferred that the recharging catheter comprises a capturing element mating said second fixation element. In a second embodiment a snare catheter is used to grab the device fixation element. The recharging catheter is then guided to the device by advancing over the snare catheter.

Additionally or alternatively, the recharging catheter preferably is dimensioned so that a distal catheter end (the catheter tip) can slip over the housing of the device. In this embodiment, the catheter has an open distal end with an inner diameter that is slightly larger than the outer diameter of the housing of the device.

In such embodiment, recharging is achieved by placing a catheter-based coil over the external housing of the device. The recharging source coil which resides within the distal end of the catheter forms a solenoid at the catheter's bore. This embodiment provides the shortest charging times as it results in the source and receiver coils aligning co-axially. In this embodiment the catheter can either exist as a lose fitting architecture that can easily slip over both the implanted device and any tissue that has encapsulated it or it could provide a tighter fit in contexts where the in-growth is either managed appropriately or the distal end of the recharging catheter presents a means for cutting through unmanaged in-growth.

Alternatively, the recharging catheter presents a smaller diameter than the device's housing and thus does not fit around the full girth of the implant housing, but instead contains a mechanism for latching onto the end of the implant such that the primary and secondary coils align coaxially (though offset along that common axis). With this configuration, the recharging catheter contains either an inner tool (e.g. a mechanical capturing element) for mechanically grabbing and holding the implant, or a magnetic tool for "recapturing" the implant. As with the first embodiment, the catheter-based coil can be employed as an electromagnet for capturing the device magnetically. In an alternative embodiment, a snare catheter is used to grab the device fixation element. The recharging catheter is then guided to the device by advancing over the snare catheter.

In an embodiment wherein the recharging catheter comprises not a capturing element, the catheter would not need to attach to or surround the device, but would instead be stationed in near proximity (for example, within the same heart chamber). With such an approach, charging is less efficient than the two previously discussed methods because the coils are not co-axial. It still however presents a case that is orders of magnitude more efficient than re-charging from the body surface due to the power transfer decay as a sixth power of the distance between inductively coupled links.

Alternatives to the claimed solution are
1.) an acceptance of gross inefficiencies for power coupling between in-body implants and outside electromagnetic sources;
2.) an abandonment of recharging efforts altogether in favor high density primary cell chemistries; and
3.) device feature set minimization for reduced implant power consumption.

The invention includes the recognition that, because power falls off to the sixth power of distance. The efficiency of inductively powered systems where the implant coil diameter is ~5 mm and the distance separating the receiver and source coils is ~8 cm (or more) is typically well under 0.1%. To make matters worse, this percentage is reported for perfectly aligned coils, whereas in practice the implanted device coil may present steep angles to the nearest external surface of the body. This misalignment of the coils compounds the inefficiencies.

In the case where sources outside the body are leveraged to instate inductive charging, the affiliated efficiency penalties are anticipated to require substantial "overdriving" of the electromagnetic source and a dramatic increase in the time required to renew charging to appropriate levels. Overdriving of the transmit coil is fundamentally limited by the Specific Absorption Rate, which in the US is 1.6 W/Kg One cannot transmit beyond this limit without risk of damaging tissue through heating. Such demands could mean that dependent patients would face a need to recharge their pacemakers relatively frequently (potentially once or more per month) using holter-like devices that would be worn for extended periods. Considering the need for an increased output from the driver unit, the recharging session might even demand that the patient remain tethered to a wall outlet or, worse yet, spend frequent 24-hour spans (or longer) in clinical care facilities.

Sidestepping rechargeable power sources in light of their noted complications motivates a need for primary cell support. Unfortunately, even the highest density battery chemistries present complications for packing appropriate capacities into packages sized to support 5 year pacing life spans for in-chamber implant devices. To add further challenge, device in-growth and the constant motion of the heart present challenges for "recapturing" the implant at the end of the primary cell's battery life. Such confounding factors create serious hurdles for device extraction/replacement, whether it will be a full change-out or a battery replacement alone.

Last, device feature set reduction, while certainly an available factor for managing power consumption, can unwittingly undermine value proposition for leadless pacemaker designs. Though it is easier to support less functionality for longer spans of time, such an approach makes the devices less useful as therapy delivery vehicles. In addition, there are limited gains by limiting device functionality. In one embodiment over 50% of the battery capacity goes directly into pacing the heart, so de-featuring a device is squeezing gains only from a minority of the energy usage because the pacing output cannot be omitted.

The claimed solution to enhanced recharging support centers on the use of dedicated periodically-scheduled sessions for administering catheter-based power renewal. During said sessions, which are anticipated occurring at most once every 5 years, the patient would step into a clinical setting and receive venous-reliant delivery of a battery recharge response. The procedure would involve the insertion of a catheter into the vasculature and the subsequent routing of its distal end to the implant site of the pacer. The terminus of the catheter (that is the catheter tip) would present a charging coil to the in-body device (that is the heart stimulation and/or monitoring device). Due to the electromagnetic coupling efficiencies achieved via the close proximity of the recharging source and the receiving coil on the pacemaker, it is expected that a rapid recharge process could ensue wherein the patient would achieve complete power source renewal in a matter of, at most, a few hours.

The optimal method for realizing the approach presented in this document, centers on the development of a catheter-based coil structure stationed in the terminus of the recharging catheter and an affiliate small-frame implant-based receiver coil in the body of the device. The catheter-based source coil, after being fed through the vasculature, would offer a means for delivering close proximity inductive recharging capabilities by mitigating a vast majority of the absorption response endemic to recharging strategies that leverage coupling at a distance and deep-body electromagnetic penetration dynamics. It is anticipated that an electrophysiologist would insert the catheter, potentially from a femoral implantation site (though others might be considered), and steer it into position such that it either latches onto the back end of the pacer or, worst case, is simply parked close to the device. Alternatively, a snare catheter is used to capture the back end of the device, and then the recharging catheter is advanced over the snare catheter to the back end of the device. After appropriately positioning the charging catheter, the patient would either be connected to charging equipment in the catheter laboratory and subjected to a recharging procedure or they would be ushered to a an out of lab monitoring station where longer duration recharging could ensue. In either case the terminus of the charging catheter located external to the body would operate as the injection port for driving power into the delivery coil. After a full recharge session, the electrophysiologist would remove the catheter and the patient would have renewed pacemaker support for another number of years.

The above and other aspects, features and advantages of the present invention will be more apparent from the following more particular description thereof, presented in conjunction with the following drawings, wherein:
- Fig. 1:: is a cross-sectional view of a leadless heart simulation and/or monitoring device placed within a distal end of a catheter;
- Fig. 2:: is a side-elevation view of the embodiment of Fig. 1;
- Figs. 3a and b:: illustrate the operation of the embodiment of Figs. 1 and 2;
- Figs. 4a and b:: disclose an alternative embodiment;
- Figs 5a and b:: illustrate a further alternative embodiment;
- Fig. 6:: is a diagrammatic representation of an embodiment of an leadless pacemaker with a fixation element to fix the leadless pacemaker to an implantation tool;
- Figs. 7 and 8:: disclose an alternative embodiment of a catheter for recharging a battery of an leadless pacemaker;

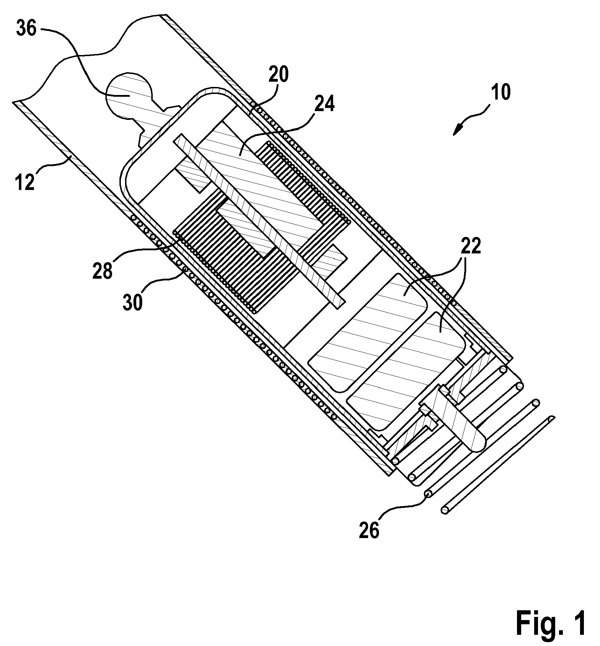

In Fig. 1, a leadless heart stimulation and/or monitoring device 10 is placed within a distal end of a catheter 12. In the embodiment of figure 1, the leadless heart stimulation and/or monitoring device 10 is a leadless pacemaker.

The heart stimulation and/or monitoring device has a sealed housing 20 that included a power supply comprising a rechargeable battery 22. Battery 22 may be composed of one or more battery cells. Housing 20 further includes electric components 24 that are operatively connected to batteries 22.

At a distal end of the heart stimulation and/or monitoring device 10, a helical screw 26 is arranged that can be screwed into heart tissue in order to fix the heart stimulation and/or monitoring device to myocardium 16 (heart tissue). Screw 26 thus serves as a first fixation element. Screw 26 may in some embodiments made from electrically conductive material so as to establish an electric contact to the myocardium after implantation and serve as sensing and/or stimulation electrode.

Batteries 22 are rechargeable. For recharging batteries 22, the device's power supply comprises an implant-based coil 28 that is dimensioned and arranged so as to receive electric power via an alternating magnetic or electro-magnetic field. Implant-based coil 28 is connected to batteries 22 via a battery charging circuit that is part of the electric components 24.

The distal end of catheter 12 is provided with a catheter-based coil 30 that can generate and emit an alternating magnetic or electromagnetic field. Implant-based coil 28 and catheter-based coil 30 are tuned so as to allow an efficient transfer of energy from coil 30 to coil 28.

Catheter-based coil 30 is electrically connected to an external energy source (not shown) at least when in use. Electric leads (not shown) providing such electric connection are integrated in catheter 12 and allow to supply power to catheter-based coil 30.

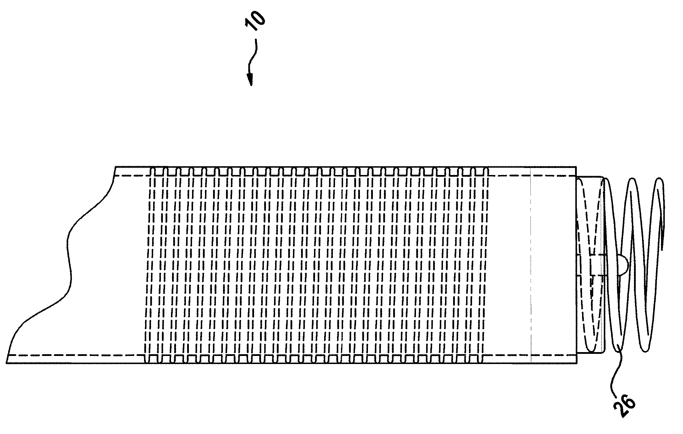

Fig. 2 is a side-elevation view of the distal part (tip) of catheter 12 and helical screw 26 of the implantable leadless pacemaker 10.

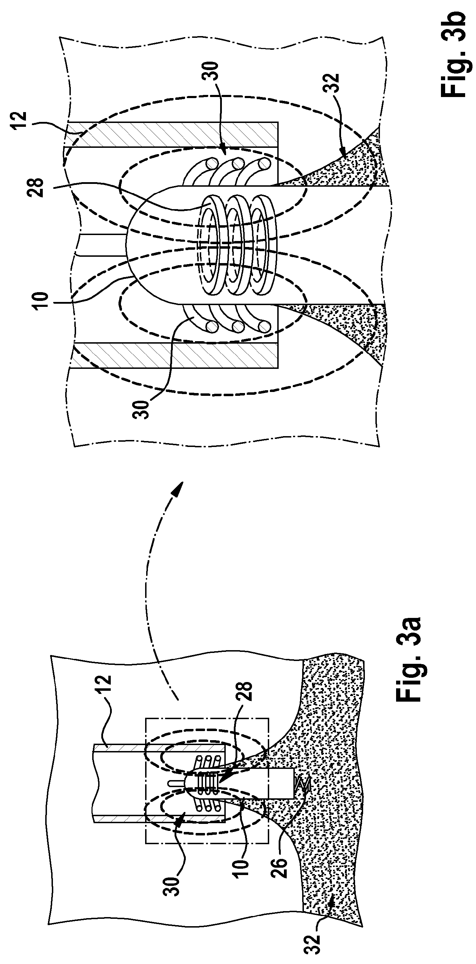

Figs. 3a and 3b illustrate the operation of the embodiment from Figs. 1 and 2.

According to this embodiment, recharging is achieved by placing the catheter-based coil 30 over the external housing 20 of the device 10. The catheter-based recharging source coil 30 which resides within the distal end of the catheter 12 forms a solenoid at the catheter's bore. In another embodiment the coil is embedded in the sidewall of the catheter. This embodiment provides the shortest charging times as it results in the source and receiver coils 30 and 28 aligning co-axially. In this embodiment the catheter 12 can either exist as a lose fitting architecture that can easily slip over both the implanted device 10 and any tissue 32 that has encapsulated it or it could provide a tighter fit in contexts where the in-growth is either managed appropriately or the distal end of the recharging catheter presents a means for cutting through unmanaged in-growth. In the case that in-growth is appropriately managed and a tighter diameter catheter tip is used, the catheter -based charging coil 30 could potentially be used to "recapture" the exposed portion of the implant via electromagnetic attraction.

Potential disadvantages of the embodiment of Figs. 1 to 3 are that it requires a large diameter catheter tip (~22 French corresponding to 7,3mm) to appropriately accommodate the implant (~19 French corresponding to 6,3 mm) and potentially its encapsulating tissue 32. These restrictions can result in increased complication risks.

It is noted that in Fig. 3a for purposes of clarity a contoured geometry of tissue 32 within ventricle not explicitly shown. 'Recapturing' the device by the recharging catheter is critical for this embodiment to work properly. With a coil present, the recharging catheter 12 can operate as an electromagnet to enable aligning and capturing the implantable device.

In order to keep the outer and thus the inner dimensions of the distal end of catheter 12 as small as possible and thus close to the outer diameter of the implantable device, recharging according to the embodiment of Figs. 1 to 3 demands the ability to either:
1.) Prevent in-growth from covering the receiver coil
2.) Have a mechanism at the tip of the catheter to cut through in-growth
   or
3.) Accept that in-growth will occur and conduct recharging processes across the tissue covering.

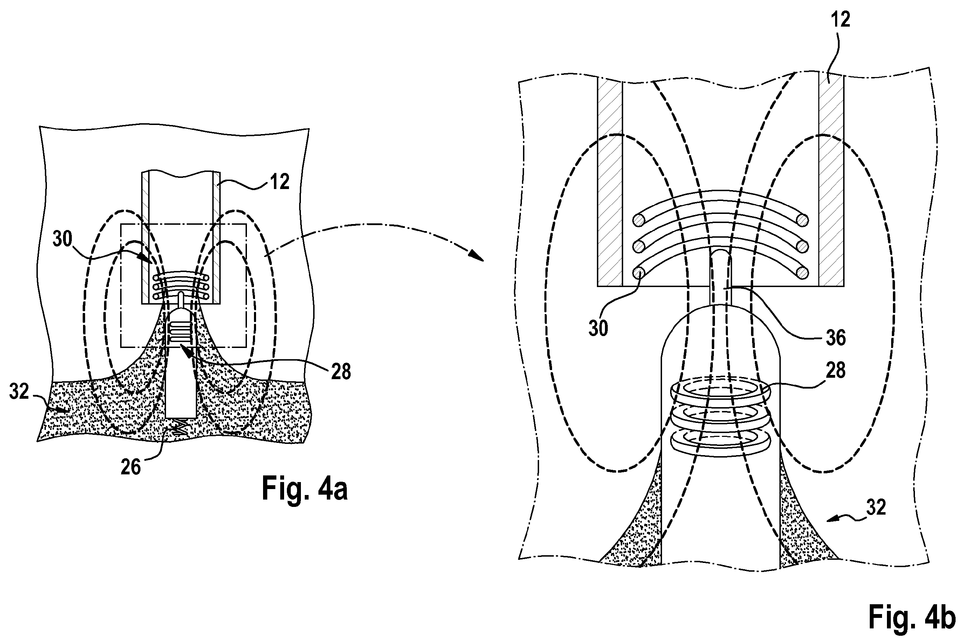

Figs. 4a and 4b illustrate a second embodiment, wherein the recharging catheter 12' presents a smaller diameter (for example, 10 French, that is 3,3mm) and does not fit around the full girth of the implant housing 20, but instead contains a mechanical capturing element for latching onto a second fixation element 36 at the proximal end of the implantable device 10 such that the primary and secondary coils 30 and 28 align coaxially (though offset along that common axis). With this configuration, the recharging catheter 12 contains either an inner tool for mechanically grabbing and holding the implant, or a magnetic tool for "recapturing" the device. To achieve the latter, catheter-based coil 30 can be employed to serve as an electromagnet for capturing the device magnetically

Similar to Fig. 3a), Fig. 4a) is not explicitly showing contoured geometry of tissue within the ventricle. As in the embodiment of Figs. 1 to 3 that leverages full-device overlap by the recharging catheter electromagnetic 'recapture' could be employed.

The type of recharging that is illustrated in Fig. 4 avoids the need to manage or cut through in-growth tissue to interface the implant. Advantageously, a catheter with a smaller diameter catheter tip leading to a less invasive process for delivering recharging can be used compared to the case where the catheter tip fully overlaps the outer housing 20 of the implant 10. Flux linkage is less robust in this approach compared to full-device overlap but is likely a worthwhile tradeoff considering gains for:
1.) Avoiding a need to cut-through or manage in-growth and
2.) Minimizing the invasiveness of the recharging.

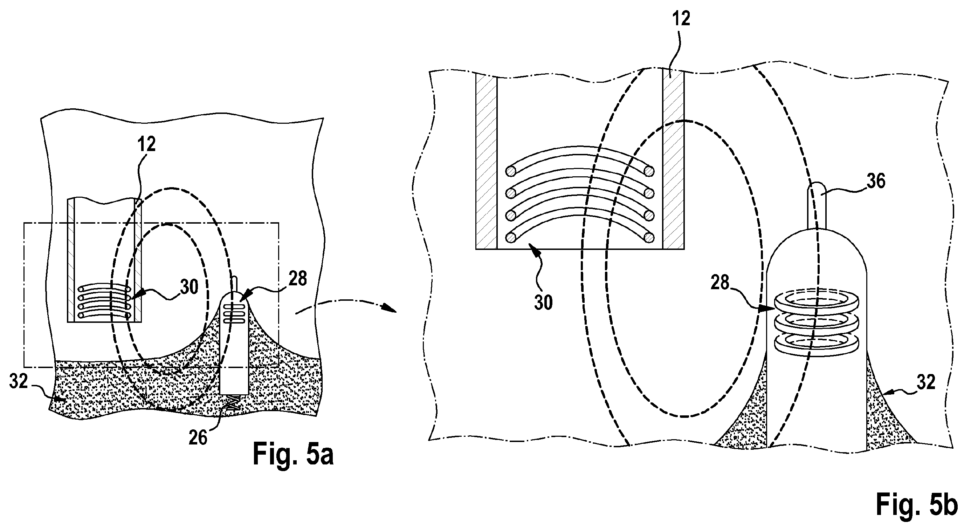

Figs. 5a and 5b illustrate yet another embodiment of this invention that would again leverage a smaller diameter recharging catheter (for example, 10 French.). In this configuration, the recharging catheter 12 would not need to attach to or surround the implant 10, but would instead be stationed in near proximity (for example, within the same heart chamber). With such an approach, charging is less efficient than the two previously discussed methods because the coils 28 and 30 are not co-axial. It still however presents a case that is orders of magnitude more efficient than re-charging from the body surface due to the power transfer decay as a 6^{th} power of the distance between inductively coupled links.

Again, in Fig. 5a) contoured geometry of tissue within the ventricle is not explicitly shown (for clarity). 'Recapture' is not essential for this embodiment. It could potentially simplify catheter design as no electro-magnet would be needed or used in the tip.

As can be taken from Fig. 5b), device recapture is not used in this embodiment.

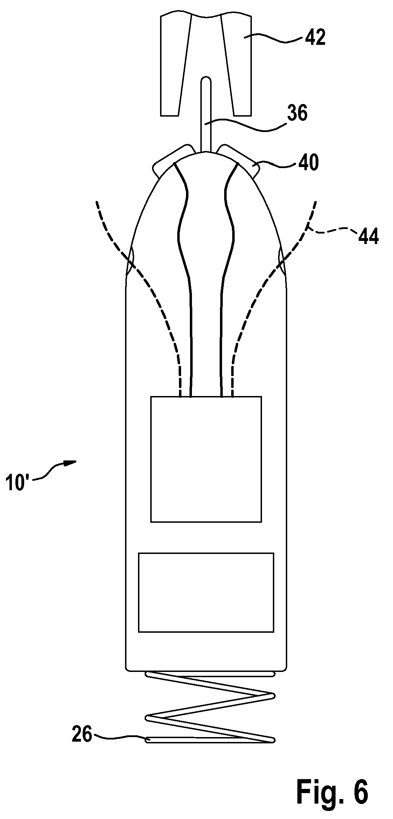

In the embodiment shown in Figure 6 the leadless pacemaker 10 has a first fixation element (helical screw 26) on its distal side to fix the leadless pacemaker 10 to the heart tissue. The first fixation element 26 in this embodiment is a helical screw 26 that is screwed into the heart tissue 32. In alternative embodiments, other fixation elements are provided that fix alone or in combination the leadless pacemaker in it's implanted position.

The leadless pacemaker 10 further has a second fixation element 36 on its proximal side to fix the leadless pacemaker 10 on the implantation tool, which is in this embodiment an introduction catheter but could as well be a recharging catheter. The second fixation element 36 connects the leadless pacemaker to the catheter. According to the embodiment of Figure 6, the second fixation element 36 may provide at least one, preferably two electrical contacts 40 that provide via corresponding contacts 42 in the catheter a communicative connection between the leadless pacemaker 10 and an external programming device (not shown) for the leadless pacemaker 10. The second fixation element 36 is in one embodiment a substantial flat extension of the proximal device housing 20 having electrical contacts 40 that are releasable clamped by the catheter. The clamps of the catheter also have electrical contacts 42 that connect to the leadless pacemaker 10 when the leadless pacemaker 10 is clamped by the catheter and provide via wire connection to the external programmer. This system allows for programming the leadless pacemaker 10 during the implantation process without the need of an inductive or radio frequency communication link for programming.

In an alternative embodiment also shown in Figure 6 the wires in the implantation tool are connected directly to the leadless pacemaker and formed as isolated break away wires 44 having predetermined breaking points close to the leadless pacemaker. In this embodiment, alternative embodiments of the second fixation elements are possible as described above in the embodiment of Figures 1 to 3. This system allows for programming the leadless pacemaker 10' during the implantation process without the need of an inductive or radio frequency communication link for programming. Once the leadless pacemaker 10' is implanted and programmed, the second fixation element 36 is released and by moving the catheter used as implantation tool back the wires break at the predetermined breaking points. The force to break the wires is less than the force to fix the implant provided by the first fixation element 26. In a further embodiment the remaining parts of the wires are connected to piezo elements that are used for harvesting of electrical energy by their movement to power the leadless pacemaker.

For the embodiment shown in Figures 1 to 3 the introduction tool is a sheath having an open distal end and an inner lumen that provides the second fixation element to fix the leadless pacemaker on its proximal side for implantation. The first fixation element on the distal side of the leadless pacemaker may be the same as described in the embodiment of Figure 6. In embodiment of Figures 1 to 3, catheter-based coil 30 may serve as a telemetry coil that is imbedded in the introducer (catheter) sheath as shown in detail in Figure 1 and used to communicate and power the leadless pacemaker 10 that resides during implantation and during recharging in the area within the catheter-based coil, which would also have a telemetry coil built axially into it. During implantation the device could be powered through the coil and programmed at the same time. This would require no power of the implants power source to program the device.

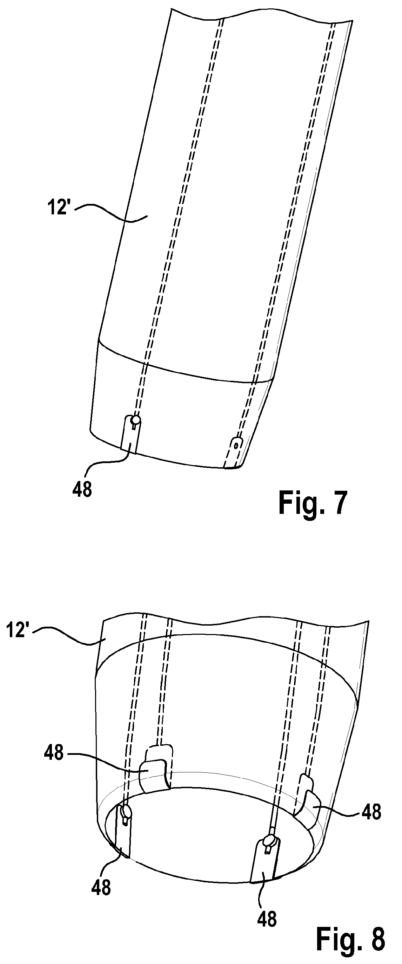

Figs. 7 and 8 show details of an additional embodiment with additional electrodes 48 for sensing electrical signals of the tissue. The additional electrodes 48 are provided on the distal end of the catheter sheath as shown in Figure 7 and Figure 8. The electrodes 48 are electrically connected to the programmer during implantation.

For example during implantation of a leadless pacemaker the catheter is forwarded up to the cardiac tissue and via the electrodes 48 of the catheter sheath electrical cardiac signals are sensed and displayed on the programmer. The implanting physician can decide whether the position is appropriate for final implantation or not and replace the distal end of the catheter if necessary. Once the final position of for implantation is found the leadless pacemaker located within the catheter sheath is forwarded and fixed, the second fixation element at the distal side of the leadless pacemaker is released and the sheath is removed.

In an alternative embodiment of the second fixation element for fixing the proximal side of the leadless pacemaker an inflatable balloon is located within the catheter sheath near the distal end of the catheter. The leadless pacemaker in this embodiment is formed at its proximal end such that the proximal end of the implant deforms the deflated balloon on introduction into the catheter sheath from the distal end of the sheath but allows the balloon to grip the proximal end of the leadless pacemaker when inflated. In a preferred embodiment the proximal end of the leadless pacemaker, as shown in figure 1 extends substantial cylindrically to a ball (which is part of the second fixation element 36) with a diameter sufficiently small to deform the deflated balloon such that the balloon, once inflated fixes the leadless pacemaker sufficiently for implantation.

The inductive recharging strategy presented in this disclosure offers a realistic avenue for dramatically extending the functional lifespan of leadless pacemakers. As most pacer designs interface with the myocardium via implant sites internal to the heart, robust anchoring methods prove essential. Device dislodgement in such contexts would knowingly lead to pulmonary embolisms (on the right-side of the heart) and/or stroke (on the left). Unfortunately, this need for robust anchoring combined with device in-growth, implant depth, and the constant contractions and blood flow native to the implant environment, present serious challenges for renewed/continual power support. The electromagnet absorption response of the human body enforces weak coupling for inductive charging sources stationed outside of the body. By directly recharging using a close-proximity catheter our approach cuts recharging times to manageable levels, trading off short-term procedure-affiliated discomforts for dramatic gains in device support and longevity. Compared to efforts that rely upon primary cell chemistries, our approach additionally overcomes periodic device replacement needs, avoiding the complications associated with implant recapture and explantation processes. Last, device feature set minimization can be driven to a reduced extent by power source management and more so via device sizing and heart geometry considerations.

## Claims

1. Leadless heart stimulation and/or monitoring system comprising an device having
- a sealed housing,
- one or more electrodes configured to electrically contact heart tissue when in use,
- electric components arranged within the housing, said electric components at least in part being operationally connected to said at least one electrode, said electric components including a power supply for providing power supply to said electric components,
- said power supply including a rechargeable battery
- said power supply further comprising an implant-based coil that is configured to receive electric power via a tuned alternating magnetic or electro-magnetic field,
said system further comprising a recharging catheter having a distal end and a catheter-based coil arranged at or near said distal end,
said catheter being dimensioned to be introduced into a heart chamber,
said catheter-based coil being tuned to said implant-based coil,
**characterized in that**,
the device comprises a second fixation element that is arranged at a proximal end and configured to be latched by a capturing element of a catheter.

2. Leadless heart stimulation and/or monitoring system according to claim 1, wherein the device comprises a first fixation element at the distal end to anchor the device in heart tissue.

3. Leadless heart stimulation and/or monitoring system according to claim 1 or 2, wherein the electric components comprise a battery recharging circuit that is electrically connected to both, the implant-based coil and the rechargeable battery of the power supply.

4. Leadless heart stimulation and/or monitoring system according to one of claims 1 to 3, wherein the housing has a diameter of less than 8 mm.

5. Leadless heart stimulation and/or monitoring system according to claim 1, wherein said recharging catheter comprises a capturing element mating said second fixation element.

6. Leadless heart stimulation and/or monitoring system according to one of claims 1 to 5, wherein said recharging catheter is dimensioned so that a distal catheter end can slip over the housing of the device.

7. Leadless heart stimulation and/or monitoring system according to one of claims 1 to 6, wherein the catheter-based coil is configured to act as an electro-magnet for capturing the device.

## Patentansprüche

1. Leitungsloses System zur Herzstimulation und/oder -überwachung, das eine Vorrichtung aufweist mit
- einem abgedichteten Gehäuse,
- einer oder mehr Elektroden, die konfiguriert sind, um im Gebrauch Herzgewebe elektrisch zu kontaktieren,
- elektrischen Komponenten, die in dem Gehäuse angeordnet sind, wobei die elektrischen Komponenten wenigstens teilweise funktionell mit der genannten wenigstens einen Elektrode verbunden sind, wobei die genannten elektrischen Komponenten eine Energieversorgung zum Bereitstellen einer Energieversorgung zu den genannten elektrischen Komponenten beinhalten,
- wobei die genannte Energieversorgung eine wiederaufladbare Batterie beinhaltet,
- wobei die genannte Energieversorgung ferner eine implantatbasierte Spule aufweist, die zum Empfangen von elektrischer Energie über ein abgestimmtes wechselndes magnetisches oder elektromagnetisches Feld konfiguriert ist,
wobei das genannte System ferner einen Aufladekatheter mit einem distalen Ende und einer katheterbasierten Spule, die an oder nahe dem genannten distalen Ende angeordnet ist, aufweist,
wobei der genannte Katheter zum Einführen in eine Herzkammer dimensioniert ist,
wobei die genannte katheterbasierte Spule auf die genannte implantatbasierte Spule abgestimmt ist,
**dadurch gekennzeichnet, dass**
die Vorrichtung ein zweites Fixierungselement aufweist, das an einem proximalen Ende angeordnet ist und konfiguriert ist, um von einem Fangelement eines Katheters arretiert zu werden.

2. Leitungsloses System zur Herzstimulation und/oder -überwachung nach Anspruch 1, wobei die Vorrichtung ein erstes Fixierungselement am distalen Ende zum Verankern der Vorrichtung im Herzgewebe aufweist.

3. Leitungsloses System zur Herzstimulation und/oder -überwachung nach Anspruch 1 oder 2, wobei die elektrischen Komponenten eine Batterieaufladeschaltung aufweisen, die elektrisch sowohl mit der implantatbasierten Spule als auch der wiederaufladbaren Batterie der Energieversorgung verbunden ist.

4. Leitungsloses System zur Herzstimulation und/oder -überwachung nach einem der Ansprüche 1 bis 3, wobei das Gehäuse einen Durchmesser von weniger als 8 mm hat.

5. Leitungsloses System zur Herzstimulation und/oder -überwachung nach Anspruch 1, wobei der genannte Aufladekatheter ein Fangelement hat, das mit dem genannten zweiten Fixierelement zusammenpasst.

6. Leitungsloses System zur Herzstimulation und/oder -überwachung nach einem der Ansprüche 1 bis 5, wobei der genannte Aufladekatheter so dimensioniert ist, dass ein distales Katheterende über das Gehäuse der Vorrichtung aufgeschoben werden kann.

7. Leitungsloses System zur Herzstimulation und/oder -überwachung nach einem der Ansprüche 1 bis 6, wobei die katheterbasierte Spule konfiguriert ist, um als Elektromagnet zum Fangen der Vorrichtung zu wirken.

## Revendications

1. Système sans fil de stimulation cardiaque et/ou de surveillance comprenant un dispositif ayant
- un boîtier étanche,
- une ou plusieurs électrodes configurées pour être électriquement en contact avec le tissu cardiaque en cours d'utilisation,
- des composants électriques disposés dans le boîtier, lesdits composants électriques étant au moins en partie connectés de manière fonctionnelle à ladite au moins une électrode, lesdits composants électriques comprenant une alimentation électrique pour fournir de l'énergie électrique auxdits composants électriques,
- ladite alimentation électrique comprenant une batterie rechargeable
- ladite alimentation électrique comprenant une bobine à la base de l'implant qui est configurée pour recevoir l'énergie électrique via un champ électromagnétique ou magnétique alternatif synchronisé,
ledit système comprenant en outre un cathéter de rechargement avec une extrémité distale et une bobine à la base du cathéter placée à l'extrémité distale ou à proximité,
ledit cathéter étant dimensionné pour être introduit dans une cavité cardiaque,
ladite bobine à la base du cathéter étant synchronisée avec ladite bobine à la base de l'implant,
**caractérisé en ce que**
le dispositif comprend un second élément de fixation qui est disposé à une extrémité proximale et qui est configuré pour être verrouillé par un élément de capture du cathéter.

2. Système sans fil de stimulation cardiaque et/ou de surveillance selon la revendication 1, dans lequel le dispositif comprend un premier élément de fixation à l'extrémité distale pour ancrer le dispositif dans le tissu cardiaque.

3. Système sans fil de stimulation cardiaque et/ou de surveillance selon les revendications 1, ou 2, dans lequel les composants électriques comprennent un circuit de rechargement de la batterie qui est connecté électriquement à la fois à la bobine à la base de l'implant et à la batterie rechargeable de l'alimentation électrique.

4. Système sans fil de stimulation cardiaque et/ou de surveillance selon l'une des revendications 1 à 3, dans lequel le boîtier a un diamètre de moins de 8 mm.

5. Système sans fil de stimulation cardiaque et/ou de surveillance selon la revendication 1, dans lequel ledit cathéter de rechargement comprend un élément de capture pour s'accoupler avec ledit second élément de fixation.

6. Système sans fil de stimulation cardiaque et/ou de surveillance selon l'une des revendications 1 à 5, dans lequel ledit cathéter de rechargement est dimensionné de façon à ce qu'une extrémité distale du cathéter puisse glisser sur le boîtier du dispositif.

7. Système sans fil de stimulation cardiaque et/ou de surveillance selon l'une des revendications 1 à 6, dans lequel la bobine à la base du cathéter est configurée pour agir comme un électro-aimant afin de capturer le dispositif.
